# EUROPEAN PATENT APPLICATION

(11) **EP 4 410 769 A1**
(43) Date of publication of application: **07.08.2024**
(21) Application number: 22876507.9
(22) Date of filing: 29.09.2022
(51) Int. Cl.: C07C 45/85, C07B 61/00, C07C 45/36, C07C 49/675

(54) **METHOD FOR PRODUCING FLUORENONE**

(30) Priority: 01.10.2021 JP 2021162749
(71) Applicant: MITSUBISHI GAS CHEMICAL COMPANY, INC., Chiyoda-ku Tokyo 100-8324 (JP)
(72) Inventor: SUMI, Hiroki, Kurashiki-shi, Okayama 712-8525 (JP); WATANABE, Yuki, Kurashiki-shi, Okayama 712-8525 (JP); FUJITA, Hideaki, Kurashiki-shi, Okayama 712-8525 (JP); NAKAMURA, Goh, Kurashiki-shi, Okayama 712-8525 (JP); KUMANO, Tatsuyuki, Kurashiki-shi, Okayama 712-8525 (JP)
(74) Representative: Vossius & Partner Patentanwälte Rechtsanwälte mbB
(86) International application number: PCT/JP2022/036595
(87) International publication number: WO 2023/054644

(57) **Abstract**

Provided is a method for producing fluorenone comprising an oxidation step of oxidizing fluorene in the presence of an aliphatic carboxylic acid having 2 to 3 carbon atoms, a metal catalyst, a bromine compound, and oxygen, and a phosphoric acid treatment step of bringing an oxidation reaction mixture into contact with phosphoric acid in the order indicated.

## Description

### Technical Field

The present invention relates to a method for producing fluorenone.

### Background Art

Fluorenone is used as a starting material or intermediate for chemicals, resins, etc. Specifically, fluorenone is a very useful compound as a starting material for electrophotographic photoreceptors, a starting material for colors, and a starting material for optical resins.

As a method for producing fluorenone, a method of oxidizing fluorene is performed, and a production method by liquid phase oxidation using an oxygen-containing gas such as air has been developed.

For example, in PTL1, for the purpose of producing fluorenones at a high yield, a method for producing fluorenones including oxidizing fluorenes with molecular oxygen in an organic solvent in the presence of a phase transfer catalyst and a solid alkali metal hydroxide is disclosed.

In PTL2, for the purpose of producing fluorenone at a high yield, a method of reacting a dimethyl sulfoxide solution of fluorene with oxygen molecules in the presence of a small amount of alkali metal hydroxide is disclosed.

In PTL3, for the purpose of easily and economically producing diallyl ketone at a high yield, a method of reacting an aromatic compound with oxygen molecules to produce a diallyl ketone using a lower saturated aliphatic monocarboxylic acid as solvent and a heavy metal as oxidation catalyst, a so-called Amoco MC method, is disclosed, and a method for producing fluorenone is disclosed as an example thereof.

Further, in PTL 4, as a method for producing high-purity fluorenone with removal of impurities in fluorenone, a method including producing derivatives having a difference in solubility, boiling point or molecular weight from fluorenone through derivatization of impurities in fluorenone and separating these derivatives from fluorenone is disclosed.

### Citation List

### Patent Literature

PTL1: JP 2007-182399 A
PTL2: US 3875237 B
PTL3: US 3038940 B
PTL4: JP 2004-115390 A

### Summary of Invention

### Technical Problem

According to PTL 4, an embodiment of the method for producing high-purity fluorenone includes heating fluorenone containing impurities with an acid catalyst and separating the produced insoluble matter for removal. However, in specific examples in PTL 4, a sample of high-purity fluorenone with addition of only specific impurities is used. Therefore, application of the method to industrial production by means of continuous operation with use of various solvents and catalysts as described above has been difficult. Further, according to PTL 3, although fluorenone is further purified by recrystallization from benzene and hexane, it has been difficult to continuously produce fluorenone in large quantities by this method.

Moreover, in actual industrial production, the resulting fluorenone may become turbid, which results in a poor appearance. Accordingly, enhancement of transparency of the product is also required.

Accordingly, an object of the present invention is to provide a production method capable of industrially obtaining fluorenone having high purity and high transparency, with efficient removal of impurities.

### Solution to Problem

As a result of extensive study by the present inventors, it has been found that the problem can be solved by oxidizing fluorene in the presence of a specific solvent, a metal catalyst and a bromine compound, and then bringing an oxidation reaction mixture into contact with phosphoric acid.

The present invention relates to the following items [1] to [11].
[1] A method for producing fluorenone comprising an oxidation step of oxidizing fluorene in the presence of an aliphatic carboxylic acid having 2 to 3 carbon atoms, a metal catalyst, a bromine compound, and oxygen, and a phosphoric acid treatment step of bringing an oxidation reaction mixture into contact with phosphoric acid in the order indicated.
[2] The method for producing fluorenone according to item [1], wherein the treatment temperature in the phosphoric acid treatment step is 140 to 350°C.
[3] The method for producing fluorenone according to item [1] or [2], wherein the concentration of phosphoric acid in the phosphoric acid treatment step is 3000 ppm by mass or more relative to the raw material fluorene.
[4] The method for producing fluorenone according to any one of items [1] to [3], wherein the treatment time of the phosphoric acid treatment step is 20 minutes or more.
[5] The method for producing fluorenone according to any one of items [1] to [4], further comprising a distillation step after the phosphoric acid treatment step.
[6] The method for producing fluorenone according to any one of items [1] to [5], wherein the metal catalyst is at least one selected from the group consisting of a cobalt catalyst, a manganese catalyst, a zirconium catalyst, a cerium catalyst, and a nickel catalyst.
[7] The method for producing fluorenone according to any one of items [1] to [6], wherein the aliphatic carboxylic acid is acetic acid.
[8] The method for producing fluorenone according to any one of items [1] to [7], wherein oxygen is supplied by introducing air in the oxidation step.
[9] The method for producing fluorenone according to any one of items [1] to [8], wherein the content of dibenzofulvene contained in the resulting fluorenone is 50 ppm by mass or less.
[10] The method for producing fluorenone according to any one of items [1] to [9], wherein a 20% (w/w) acetone solution of the resulting fluorenone with a thickness of 10 mm has a haze of 0.60 or less.
[11] The method for producing fluorenone according to any one of items [1] to [10], wherein the resulting fluorenone with a thickness of 25 mm has an a value of -10.30 or less at 120°C.

### Advantageous Effects of Invention

According to the present production method, impurities can be efficiently removed, and fluorenone having high purity and high transparency can be obtained industrially. In particular, by liquid phase oxidation of fluorenone, specifically with use of Amoco MC method, fluorenone having high purity and high transparency can be produced continuously.

### Description of Embodiments

A method for producing fluorenone of the present invention comprises an oxidation step of oxidizing fluorene in the presence of an aliphatic carboxylic acid having 2 to 3 carbon atoms, a metal catalyst, a bromine compound, and oxygen, and a phosphoric acid treatment step of bringing an oxidation reaction mixture into contact with phosphoric acid in the order indicated.

Here, the oxidation reaction mixture is an oxidation reaction mixture obtained in the oxidation step or an oxidation reaction mixture obtained in the solvent removal step for removing the aliphatic carboxylic acid having 2 to 3 carbon atoms after the oxidation step.

In the present invention, a large amount of fluorenone can be obtained with a small amount of solvent at high productivity. Fluorenone is obtained by Amoco MC method having a high production efficiency, and the resulting fluorenone is brought into contact with phosphoric acid without performing further treatments such as purifying, so that the purity and the transparency of fluorenone can be enhanced. Further, fluorenone excellent in appearance without dullness can be obtained.

The production method of the present invention is described in detail as follows.

### [Oxidation step]

In the method for producing fluorenone of the present invention, first, fluorene is oxidized. By the oxidation step, fluorene can be oxidized to produce fluorenone as a main product.

In the oxidation step in the method for producing fluorenone of the present invention, fluorene is oxidized in the presence of an aliphatic carboxylic acid having 2 to 3 carbon atoms, a metal catalyst, a bromine compound, and oxygen, so that an oxidation reaction mixture containing fluorenone as a main product is obtained.

Before introducing oxygen, a pretreatment step of heating fluorene in the presence of a lower aliphatic carboxylic acid, a bromine compound and a metal catalyst may be performed.

### <Aliphatic carboxylic acid having 2 to 3 carbon atoms>

The aliphatic carboxylic acid used in the present step is an aliphatic carboxylic acid having 2 to 3 carbon atoms, and more preferably an aliphatic carboxylic acid having 2 carbon atoms.

Preferably, specific examples of the aliphatic carboxylic acid include at least one selected from the group consisting of acetic acid and propionic acid, and acetic acid is more preferred. In the case of using acetic acid, water and acetic acid may be mixed to prepare a mixed solution in advance for use as described later, or only acetic acid may be used.

It is preferable to use the aliphatic carboxylic acid because the activity of the catalyst may be enhanced.

The amount of the aliphatic carboxylic acid used in the oxidation step is preferably 10 to 1000 parts by mass, more preferably 50 to 400 parts by mass, still more preferably 70 to 200 parts by mass, and furthermore preferably 80 to 100 parts by mass relative to 100 parts by mass of fluorene.

With an amount of the aliphatic carboxylic acid equal to or more than the lower limit, the viscosity may be made appropriate for easy handling, and the heat of reaction may be controlled to prevent the reactor temperature from rising. Further, with an amount of the aliphatic carboxylic acid equal to or less than the upper limit, the production efficiency is excellent and the economy is also excellent.

One aliphatic carboxylic acid may be used alone, or two or more may be used in combination.

### <Metal catalyst>

The metal catalyst used in the present step is preferably at least one selected from the group consisting of a transition metal catalyst and a rare earth metal catalyst, more preferably a transition metal catalyst.

Preferably, examples of the specific transition metal catalyst include at least one selected from the group consisting of a cobalt catalyst, a manganese catalyst, a zirconium catalyst, a cerium catalyst, and a nickel catalyst, and more preferably, examples thereof include at least one selected from the group consisting of a cobalt catalyst and a manganese catalyst. Still more preferably, both of a cobalt catalyst and a manganese catalyst are used.

As described above, the specific metal catalyst used in the present step is preferably at least one selected from the group consisting of a cobalt catalyst, a manganese catalyst, a zirconium catalyst, a cerium catalyst, and a nickel catalyst, and more preferably at least one selected from the group consisting of a cobalt catalyst and a manganese catalyst. Still more preferably, both of a cobalt catalyst and a manganese catalyst are used.

A metal catalyst may be used in the form of a salt, an elemental metal, an oxide, a hydroxide, or the like. The metal catalyst used in the present step is preferably a salt, more preferably an aliphatic carboxylate, still more preferably a lower aliphatic carboxylate, and furthermore preferably an acetate. In particular, at least one selected from the group consisting of cobalt acetate and manganese acetate is furthermore preferred.

It is preferable to use the metal catalyst, because fluorenone can be obtained at a high yield.

The amount of the metal catalyst used in the oxidation step in terms of metal elements is preferably 0.02 to 10 parts by mass, more preferably 0.05 to 5 parts by mass, still more preferably 0.1 to 3 parts by mass, and furthermore preferably 0.1 to 1 part by mass, relative to 100 parts by mass of fluorene.

With a catalyst concentration equal to or more than the lower limit, the reaction rate is improved and the yield is also improved. With a catalyst concentration equal to or less than the upper limit, the catalyst cost is reduced and the reaction is not adversely affected.

One metal catalyst may be used alone, or two or more may be used in combination.

### <Bromine compound>

Examples of the bromine compound used in the present step preferably include hydrogen bromide, a bromide salt, and an organic bromine compound, more preferably include at least one selected from the group consisting of hydrogen bromide and a bromide salt, and still more preferably include hydrogen bromide.

Hydrogen bromide is preferably used as an aqueous solution.

Specific examples of the bromide salt include sodium bromide, potassium bromide, and ammonium bromide.

The amount of the bromine compound in terms of bromine used in the oxidation step is preferably 0.01 to 5 parts by mass, more preferably 0.05 to 3 parts by mass, still more preferably 0.05 to 1 part by mass, and furthermore preferably 0.05 to 0.5 parts by mass, relative to 100 parts by mass of fluorene.

With an amount of the bromine compound equal to or more than the lower limit of the range, the reaction rate is improved and the yield is also improved. With an amount of the bromine compound equal to or less than the upper limit of the range, corrosion is hardly caused and an apparatus made of high-grade material is unnecessary.

One bromine compound may be used alone, or two or more may be used in combination.

### <Water>

Water may be used in the present step. It is preferable to use water because a bromine compound is easily dissolved.

The amount of water used in the oxidation step is preferably 1 to 200 parts by mass, more preferably 1 to 100 parts by mass, still more preferably 2 to 50 parts by mass, and furthermore preferably 3 to 10 parts by mass, relative to 100 parts by mass of fluorene.

With a water concentration in the range, the bromine compound may be dissolved while preventing the catalytic activity from being lowered, so that the yield may be improved.

### <Pretreatment step>

In the method for producing fluorenone of the present invention, first, a pretreatment step of heating fluorene in the presence of a lower aliphatic carboxylic acid, a bromine compound and a metal catalyst may be performed.

The amount of fluorene used in the present step is preferably 1 to 50 mass%, more preferably 2 to 40 mass%, still more preferably 3 to 30 mass%, and even more preferably 5 to 20 mass% relative to the total fluorene used as raw material in the oxidation reaction.

By controlling the amount of fluorene used in the present step to the range, increase in the molecular weight of fluorene due to side reactions and decrease in the yield associated therewith can be suppressed, which is preferable.

The fluorene used in the present step may be introduced all at once to be heated, or may be gradually and continuously introduced. Continuous introduction is preferred from the viewpoint of suppressing side reactions.

The heating temperature in the pretreatment step is preferably 160 to 250°C, more preferably 180 to 250°C, still more preferably 200 to 250°C, even more preferably 220 to 250°C, and furthermore preferably 220 to 240°C.

With a heating temperature controlled to the range, control of the reaction rate is made easy, which is preferable.

The heating time in the pretreatment step may be appropriately changed depending on the heating temperature, the amounts of the catalyst and raw materials, the sizes of the reaction container, the method of introducing the raw materials, etc., and is preferably 3 to 30 minutes, more preferably 3 to 20 minutes, still more preferably 3 to 15 minutes, and even more preferably 5 to 15 minutes.

With a heating time controlled to the range, the increase in the molecular weight of fluorene due to a side reaction and the decrease in the yield associated therewith can be suppressed, which is preferable.

### <Oxygen>

As oxygen for use in the present step, an oxygen gas may be used, or a mixed gas including an inert gas or the like may be used. The inert gas is preferably at least one selected from the group consisting of nitrogen and rare gases, more preferably nitrogen. Among the rare gases, argon is preferred. In the present step, in particular, oxygen is preferably supplied by introducing air from the viewpoint of safety and economy. Air is a mixed gas containing nitrogen and oxygen as main components.

The oxygen used in the present step is introduced such that the oxygen concentration in the exhaust gas (off-gas) discharged from a reactor during supply of starting materials is preferably 0.1 to 8 vol%, more preferably 1 to 5 vol%.

With an introduced amount of oxygen maintained within the range, the reaction may be performed safely and efficiently in a preferred manner below an explosion range of the solvent.

### <Conditions for oxidation step>

The temperature during the oxidation reaction in the present step is preferably 120 to 280°C, more preferably 160 to 260°C, and even more preferably 190 to 240°C.

With a temperature during the oxidation reaction equal to or more than the lower limit of the range, the reaction rate is improved, and with a temperature during the oxidation reaction equal to or less than the upper limit of the range, formation of by-products is suppressed to improve the yield.

The pressure during the oxidation reaction in the present step may be in a pressure range that allows the reaction solution to be kept in the liquid phase, and is preferably 0.1 to 4 MPa.

The reaction time of the oxidation reaction in the present step is preferably 0.1 to 10 hours, more preferably 0.5 to 5 hours, and still more preferably 1 to 3 hours in the case where oxygen is supplied by introducing air.

In the present step, from the viewpoint of safety, it is preferable to introduce an inert gas such as nitrogen into a reaction container containing the starting materials before introducing oxygen.

### [Solvent removal step]

The method for producing fluorenone of the present invention preferably includes a solvent removal step after the oxidation step.

In the solvent removal step in the method for producing fluorenone of the present invention, the solvent is removed from the oxidation reaction mixture obtained in the oxidation step so as to obtain an oxidation reaction mixture containing fluorenone as main component.

In the present step, aliphatic carboxylic acids having 2 to 3 carbon atoms are preferably removed, and in the case where water is used in the oxidation reaction step, water may also be removed in the present step.

In the present step, in order to increase the production efficiency, the solvent may be removed by heat distillation under reduced pressure, or the solvent may be removed by heat distillation under atmospheric pressure.

The pressure during removal of solvent in the present step is preferably 80 kPa or less, more preferably 1 to 60 kPa, and still more preferably 2 to 50 kPa.

The temperature during removal of solvent in the present step is preferably 80 to 200°C, more preferably 90 to 1 80°C, and still more preferably 100 to 150°C.

In the present step, an apparatus used for general heat distillation is used, and specific examples thereof include a simple distillation apparatus, a precision distillation apparatus, a molecular distillation apparatus, and a thin film distillation apparatus. Other than these distillation apparatuses, the solvent may be removed by using a dryer or the like.

### [Phosphoric acid treatment step]

The method for producing fluorenone of the present invention includes a phosphoric acid treatment step of bringing the oxidation reaction mixture into contact with phosphoric acid after the oxidation step. The method for producing fluorenone of the present invention includes the oxidation step and a phosphoric acid treatment step of bringing an oxidation reaction mixture into contact with phosphoric acid in the order indicated.

In the case of having a solvent removal step after the oxidation step, the method includes a phosphoric acid treatment step of bringing the oxidation reaction mixture into contact with phosphoric acid after solvent removal step.

That is, the method for producing fluorenone of the present invention is any one of: the method including an oxidation step of oxidizing fluorene in the presence of an aliphatic carboxylic acid having 2 to 3 carbon atoms, a metal catalyst, a bromine compound, and oxygen, and a phosphoric acid treatment step of bringing an oxidation reaction mixture obtained in the oxidation step into contact with phosphoric acid in the order indicated; and the method including an oxidation step of oxidizing fluorene in the presence of an aliphatic carboxylic acid having 2 to 3 carbon atoms, a metal catalyst, a bromine compound and oxygen, a solvent removal step of removing aliphatic carboxylic acids having 2 to 3 carbon atoms from the oxidation reaction mixture obtained in the oxidation step so as to obtain an oxidation reaction mixture containing fluorenone as main component, and a phosphoric acid treatment step of bringing the oxidation reaction mixture obtained in the solvent removal step into contact with phosphoric acid in the order indicated.

By including the present step in the method for producing fluorenone of the present invention, impurities can be efficiently removed, so that fluorenone having high purity and high transparency can be obtained industrially. In the present step, it is presumed that by using phosphoric acid as an acid catalyst, impurities by-produced in the oxidation reaction can be polymerized and removed from fluorenone as target product. In particular, in the present production method using a metal catalyst in the oxidation reaction, an acid catalyst other than phosphoric acid cannot remove impurities, probably because it forms a salt with the metal catalyst and does not act as catalyst. In contrast, in the present invention, impurities can be efficiently removed by using phosphoric acid even in Amoco MC method using a metal catalyst in the oxidation step, so that fluorenone having high purity and high transparency can be produced continuously. Further, since use of phosphoric acid generates no corrosive gas such as hydrogen halide, industrial use thereof is preferred. Furthermore, fluorenone excellent in appearance without dullness can be obtained.

The concentration of phosphoric acid in the phosphoric acid treatment step is preferably 3000 ppm by mass or more, more preferably 4000 ppm by mass or more, still more preferably 5000 ppm by mass or more relative to the raw material fluorene (the starting material fluorene of the oxidation step), and may be preferably 7000 ppm by mass or more relative to the raw material fluorene. The upper limit is not limited, and it can be presumed that the higher the concentration of phosphoric acid, the higher the effect of the present invention to obtain fluorenone having transparency without dullness. From the viewpoints of cost and production efficiency, the upper limit is preferably 20000 ppm by mass or less, more preferably 15000 ppm by mass or less, and in practice, may be 10000 ppm by mass or less.

The treatment temperature in the phosphoric acid treatment step is preferably 120 to 350°C, more preferably 140 to 350°C, still more preferably 150 to 350°C, even more preferably 180 to 320°C, furthermore preferably 200 to 300°C, and furthermore preferably 230 to 250°C. The treatment temperature in the phosphoric acid treatment step may be appropriately changed depending on the concentration of phosphoric acid, the amount of impurities, and the like.

The treatment time of the phosphoric acid treatment step is preferably 20 minutes or more, more preferably 30 minutes or more, and still more preferably 40 minutes or more. The upper limit is preferably 24 hours or less, though not limited. The treatment time of the phosphoric acid treatment step may be appropriately changed depending on the concentration of phosphoric acid, the amount of impurities, the treatment temperature, and the like.

In the case where the phosphoric acid concentration in the phosphoric acid treatment step is 4000 to 10000 ppm by mass, the treatment temperature is preferably 230°C to 250°C. That is, it is preferable that the phosphoric acid concentration in the phosphoric acid treatment step be 5000 to 10000 ppm by mass and the treatment temperature be 230°C to 250°C. Also, in the case where the phosphoric acid concentration in the phosphoric acid treatment step is 4000 to 10000 ppm by mass, the treatment temperature is preferably 230°C to 250°C. That is, it is also preferable that the phosphoric acid concentration in the phosphoric acid treatment step be 5000 to 10000 ppm by mass and the treatment temperature be 230°C to 250°C.

### [Distillation step]

The method for producing fluorenone of the present invention preferably includes a distillation step after the phosphoric acid treatment step .

The distillation step may be any method as long as the target fluorenone may be separated and collected. It is more preferable that the distillation step include a step of removing high boiling point components and a step of removing low boiling point components in the order indicated.

Further, in the step of removing low boiling components using a distillation column, in the case where the residence time is long at the column bottom, fluorenone may be deteriorated and colored, so it is still more preferable to further include a step of removing a coloring component.

The two-step distillation, which is a preferred embodiment, will be described below.

### (High boiling point component removal step)

First, it is preferable to remove high boiling point components. It is presumed that the high boiling point components contain various impurities and by-products, and by removing the high boiling point components at the beginning of the distillation step, it is presumed that deterioration of the target product and the increase of impurities due to decomposition of the high boiling point components may be suppressed.

The distillation temperature may be appropriately adjusted to about the boiling point of fluorenone under the pressure during distillation (boiling point at 1 atm is 342°C). For example, in the case where the distillation pressure is adjusted to 1.5 to 3.5 kPa, the distillation temperature is preferably 150 to 300°C, more preferably 160 to 250°C, still more preferably 170 to 240°C, and furthermore preferably 180 to 220°C.

In the case where a distillation column is used in the present high boiling point component removal step, a mixture containing fluorenone and low boiling point components are collected from the column top.

### (Low boiling point component removal step)

Subsequently, it is preferable that low boiling point components be removed.

The distillation temperature in the present step may be appropriately adjusted to about the boiling point of fluorenone under the pressure during distillation (boiling point at 1 atm is 342°C). For example, in the case where the distillation pressure is adjusted to 1.5 to 3.5 kPa, the distillation temperature is preferably 150 to 300°C, more preferably 160 to 250°C, still more preferably 165 to 230°C, and furthermore preferably 170 to 200°C.

In the case where a distillation column is used in the present low boiling point component removal step, high-purity fluorenone may be collected from the column bottom.

In the case where the residence time at the column bottom is long, fluorenone may be deteriorated and colored. Therefore, in order to remove coloring components, it is preferable that further distillation be performed after the present distillation step (coloring component removal step), so that high-purity fluorenone is collected from the column top.

### [Properties of the fluorenone of the present invention]

According to the production method of the present invention, impurities can be efficiently removed, and fluorenone having high purity and high transparency can be obtained industrially. Accordingly, the fluorenone obtained by the production method of the present invention (the resulting fluorenone) preferably has the following properties.

The content of dibenzofulvene contained in the fluorenone obtained by the production method of the present invention is preferably 100 ppm by mass or less, more preferably 70 ppm by mass or less, still more preferably 50 ppm by mass or less, and even more preferably 10 ppm by mass or less. With a content of dibenzofulvene of 10 ppm by mass or less, the product is sufficiently transparent with no dullness observed.

The haze of a 20% (w/w) acetone solution of fluorenone having a thickness of 10 mm obtained by the production method of the present invention is preferably 1.0 or less, more preferably 0.70 or less, still more preferably 0.60 or less, even more preferably 0.30 or less, furthermore preferably 0.20 or less, and furthermore preferably 0.15 or less. Practically, a haze of 0.20 or less is sufficiently transparent. The haze is obtained by measuring transmitted light to the light incident in the thickness direction of the acetone solution. The haze can be determined by placing a 20% (w/w) acetone solution of fluorenone in a measuring cell having a thickness of 10 mm and measuring under the measurement conditions according to JIS K7136. Specifically, the haze is determined by the method described in Examples.

The a value at 120°C of fluorenone having a thickness of 25 mm obtained by the production method of the present invention is preferably -10.30 or less, more preferably - 10.00 or less, still more preferably -11. 00 or less, and even more preferably -12.00 or less. With an a value in the range, the dullness of fluorenone is less, which is preferable. The a value corresponds to the a value of fluorenone in a molten state. The a value is obtained by measuring the transmitted light to the light incident in the thickness direction of fluorenone in a molten state. The a value can be determined by heating and melting fluorenone in a glass test tube with an inner diameter of 25 mm at 120°C, and performing the measurement under the measurement conditions according to JIS Z 8722. Specifically, the a value is obtained by the method described in Examples.

### Examples

The present invention will be specifically described based on Examples shown below, though the present invention is not limited thereto.

### [Analysis Method and Evaluation Method]

### <Method for measuring each component concentration (composition)>

In Examples 1 to 4 and Comparative Example 1, the composition (purity and content) of each component (fluorene, fluorenone, 9-fluorenyl acetate, dibenzofulvene, 9-methyl-9-fluorenol, and 9,9'-bisfluorenyl) in a reaction product after the oxidation step, the solvent removal step, the phosphoric acid treatment step and the heating step was calculated by an internal reference method using gas chromatography (internal reference: triphenylmethane).

The composition (purity and content) of fluorene and 9-fluorenones (fluorene, fluorenone and 9-fluorenyl acetate (fluorenyl acetate), dibenzofulvene, and 9-methyl-9-fluorenol) after the distillation step is an area percentage by gas chromatography.

As an impurity contained in the final product (fluorenone), dibenzofulvene was quantified. As the amount of dibenzofulvene decreases, the resulting fluorenone has higher purity, being favorably transparent without dullness. The results are shown in Table 1.

The content of dibenzofulvene in each of Example 5 and Comparative Examples 2 to 8 was also calculated by an internal standard method using gas chromatography (internal reference: triphenylmethane).

### <Evaluation of dullness (measurement of a value)>

In a glass test tube with an inner diameter of 25 mm, 20 g of fluorenone obtained in each of Examples and Comparative Examples (final product, purified fluorenone) was placed and melted with a block heater heated to 120°C. The a value was measured with a color difference meter (SE7700, manufactured by Nippon Denshoku Industries Co., Ltd.). The measurement conditions were according to JIS Z 8722. The results are shown in Table 1.

As the a value decreases, the resulting fluorenone has a higher chroma without dullness in appearance.

### <Haze>

The haze was measured as follows.

After dissolving 3 g of the fluorenone (final product, purified fluorenone) obtained in each of Examples and Comparative Examples in 12 g of acetone, the solution was placed in a measurement cell, and the haze was measured with a haze meter (COH7700, manufactured by Nippon Denshoku Industries Co., Ltd.). A quartz cell (thickness: 10 mm) was used as the measurement cell. The measurement conditions were according to JIS K7136. The results are shown in Table 1.

As the haze value decreases, the resulting fluorenone has less turbidity and better transparency.

### [Production of fluorenone]

### Example 1

### (1. Oxidation step)

Cobalt acetate tetrahydrate, manganese acetate tetrahydrate, 48 mass% hydrogen bromide aqueous solution, glacial acetic acid, and water were mixed to obtain a catalyst solution having a cobalt metal atom concentration of 0.24 mass%, a manganese metal atom concentration of 0.15 mass%, a bromine ion concentration of 0.18 mass%, an acetic acid concentration of 89.61 mass%, with the balance water (concentration of about 10 mass%).

An autoclave made of titanium with an internal volume of 500 mL having a gas discharge pipe with a reflux condenser, a gas blow pipe, a continuous starting material liquid feeding pump and a stirrer was charged with 150 g of the catalyst solution. Under nitrogen atmosphere, the pressure was increased to2.0 MPa and the temperature was raised to 230°C. 150 g of fluorene was supplied thereto in 90 minutes. The throughput was 1.3 g/min. Air was introduced after 5 minutes from the start of supply of the starting material, and the oxidation reaction was performed by adjusting the amount of air introduced such that the oxygen concentration in the off-gas was 4 vol% or less. After termination of the supply of fluorene, the absorption of oxygen ended, and the introduction of air was terminated when the oxygen concentration in the off-gas reached 8 vol%. As a result of extracting and analyzing the resulting oxidation reaction product, the composition included 0.58 mass% of fluorene, 43.49 mass% of fluorenone, 0.26 mass% of 9-fluorenyl acetate, 0.01 mass% of 9,9'-bisfluorenyl, and 0.01 mass% of 9-methyl-9-fluorenol.

### (2. Solvent removal step)

Into a 500-mL flask made of glass, 430 g of the oxidation reaction product was transferred, and simple distillation was performed over 1 hour at a heater temperature of 120°C and an internal pressure of 5 to 40 kPa, so that 210 g of acetic acid and water were distilled off. The composition of the reaction product after removal of the solvent included 0.29 mass% of fluorene, 83.52 mass% of fluorenone, 0.65 mass% of 9-fluorenyl acetate, 0.01 mass% of 9,9'-bisfluorenyl, 0.02 mass% of 9-methyl-9-fluorenol, and 0.020 mass% (200 ppm by mass) of dibenzofulvene.

### (3. Phosphoric acid treatment step)

To the reaction product after solvent removal, 5000 ppm by mass of phosphoric acid (manufactured by FUJIFILM Wako Pure Chemical Corporation) (amount relative to raw material fluorene) was added, and then the mixture was introduced into a distillation column with 9 stages to be refluxed at a pressure of 2 kPa at 245.0 to 250.0°C for 1 hour. The composition of the reaction product after refluxing included 0.02 mass% of fluorene, 83.76 mass% of fluorenone, and 0.01 mass% of 9,9'-bisfluorenyl, and fluorenyl acetate, 9-methyl-9-fluorenol, and dibenzofulvene were not included therein.

### (4. Distillation step (high boiling point component removal step))

After the phosphoric acid treatment step, extraction was started under distillation conditions set to a pressure of 2 kPa, a column top temperature of 188.0°C, and a column bottom temperature of 196.0°C. From the distillation column top, the resulting fluorenone containing low boiling point components was extracted. The composition included 0.02 mass% of fluorene, 99.73 mass% of fluorenone, and the collection rate of fluorenone by the distillation was 80.76%.

### (5. Distillation step (low boiling point component removal step))

Fluorenone containing low boiling point components obtained from the distillation column top was subjected to batch distillation to separate the low boiling point components from fluorenone using a distillation column corresponding to 9 stages. The distillation conditions were set to a pressure of 1.7 kPa, a column top temperature of 185.0 °C, and a column bottom temperature of 190.0°C. Fluorenone was obtained from the distillation column bottom. The fluorenone obtained from the bottom of the distillation column was subjected to a coloring component removal step (distillation at a pressure of 1.7 kPa and a temperature of 190°C.) to obtain purified fluorenone from the top of the distillation column. The resulting purified fluorenone had a purity of 99.98%, a distillation recovery rate of fluorenone of 72.2%, and a haze of 0.11. The purified fluorenone included 0.001 mass% of raw material fluorene, and fluorenyl acetate, 9-methyl-9-fluorenol, and dibenzofulvene were not included therein (detection limit: 0.001 mass% or less (10 ppm by mass or less)).

### Example 2

The same operation as in Example 1 was performed up to "2. Solvent removal step", so that a reaction product after solvent removal was obtained. The operations on "3. Phosphoric acid treatment step" and the followings were performed as follows.

### (3. Phosphoric acid treatment step)

To the reaction product after solvent removal, 3000 ppm by mass of phosphoric acid (manufactured by FUJIFILM Wako Pure Chemical Corporation) (amount relative to raw material fluorene) was added, and then the mixture was introduced into a distillation column with 9 stages to be refluxed at a pressure of 2 kPa at 245.0 to 250.0°C for 1 hour. The composition of the reaction product after refluxing included 0.18 mass% of fluorene, 83.15 mass% of fluorenone, and 0.02 mass% of 9,9'-bisfluorenyl, and 0.013 mass% (130 ppm by mass) of dibenzofulvene, and fluorenyl acetate and 9-methyl-9-fluorenol were not included therein.

### (4. Distillation step (high boiling point component removal step))

After the phosphoric acid treatment step, extraction was started under distillation conditions set to a pressure of 2 kPa, a column top temperature of 188.0°C, and a column bottom temperature of 196.0°C. From the distillation column top, the resulting fluorenone containing low boiling point components was extracted. The composition included 0.12 mass% of fluorene, 98.98 mass% of fluorenone, and 0.01 mass% of dibenzofulvene, and the collection rate of fluorenone by the distillation was 91.43%.

### (5. Distillation step (low boiling point component removal step))

Fluorenone containing low boiling point components obtained from the distillation column top was subjected to batch distillation to separate the low boiling point components from fluorenone using a distillation column corresponding to 9 stages. The distillation conditions were set to a pressure of 1.7 kPa, a column top temperature of 185.0 °C, and a column bottom temperature of 190.0°C. Fluorenone was obtained from the distillation column bottom. The fluorenone obtained from the bottom of the distillation column was subjected to a coloring component removal step (distillation at a pressure of 1.7 kPa and a temperature of 190°C.) to obtain purified fluorenone from the top of the distillation column. The resulting purified fluorenone had a purity of 99.92%, a distillation recovery rate of fluorenone of 61.5%, and a haze of 0.67. The purified fluorenone included 0.01 mass% of raw material fluorene, 0.006 mass%(60 ppm by mass) of dibenzofulvene, and fluorenyl acetate and 9-methyl-9-fluorenol were not included therein (detection limit: 0.001 mass% or less (10 ppm by mass or less)).

### Example 3

The same operation as in Example 1 was performed up to "2. Solvent removal step", so that a reaction product after solvent removal was obtained. Operations on "3. Phosphoric acid treatment step" and the followings were performed as follows.

### (3. Phosphoric acid treatment step)

To the reaction product after solvent removal, 4000 ppm by mass of phosphoric acid (manufactured by FUJIFILM Wako Pure Chemical Corporation) (amount relative to raw material fluorene) was added, and then the mixture was introduced into a distillation column with 9 stages to be refluxed at a pressure of 2 kPa at 245.0 to 250.0°C for 1 hour. The composition of the reaction product after refluxing included 0.049 mass% of fluorene, 89.775 mass% of fluorenone, and 0.002 mass% of 9,9'-bisfluorenyl, and fluorenyl acetate, 9-methyl-9-fluorenol, and dibenzofulvene were not included therein.

### (4. Distillation step (high boiling point component removal step))

After the phosphoric acid treatment step, extraction was started under distillation conditions set to a pressure of 2 kPa, a column top temperature of 188.0°C, and a column bottom temperature of 196.0°C. From the distillation column top, the resulting fluorenone containing low boiling point components was extracted. The composition included 0.041 mass% of fluorene and 99.282 mass% of fluorenone, and the collection rate of fluorenone by the distillation was 77.27%.

### (5. Distillation step (low boiling point component removal step))

Fluorenone containing low boiling point components obtained from the distillation column top was subjected to batch distillation to separate the low boiling point components from fluorenone using a distillation column corresponding to 9 stages. The distillation conditions were set to a pressure of 1.7 kPa, a column top temperature of 185.0 °C, and a column bottom temperature of 190.0°C. Fluorenone was obtained from the distillation column bottom. The fluorenone obtained from the bottom of the distillation column was subjected to a coloring component removal step (distillation at a pressure of 1.7 kPa and a temperature of 190°C.) to obtain purified fluorenone from the top of the distillation column. The resulting purified fluorenone had a purity of 99.89%, a distillation recovery rate of fluorenone of 73.35%, and a haze of 0.13. The purified fluorenone included 0.012 mass% of raw material fluorene, and fluorenyl acetate, 9-methyl-9-fluorenol, and dibenzofulvene were not included therein (detection limit: 0.001 mass% or less (10 ppm by mass or less)).

### Example 4

The same operation as in Example 1 was performed up to "2. Solvent removal step", so that a reaction product after solvent removal was obtained. Operations on "3. Phosphoric acid treatment step" and the followings were performed as follows.

### (3. Phosphoric acid treatment step)

To the reaction product after solvent removal, 10000 ppm by mass of phosphoric acid (manufactured by FUJIFILM Wako Pure Chemical Corporation) (amount relative to raw material fluorene) was added, and then the mixture was introduced into a distillation column with 9 stages to be refluxed at a pressure of 2 kPa at 245.0 to 250.0°C for 1 hour. The composition of the reaction product after refluxing included 0.051 mass% of fluorene, 86.830 mass% of fluorenone, and 0.120 mass% of 9,9'-bisfluorenyl, and fluorenyl acetate, 9-methyl-9-fluorenol, and dibenzofulvene were not included therein.

### (4. Distillation step (high boiling point component removal step))

After the phosphoric acid treatment step, extraction was started under distillation conditions set to a pressure of 2 kPa, a column top temperature of 188.0°C, and a column bottom temperature of 196.0°C. From the distillation column top, the resulting fluorenone containing low boiling point components was extracted. The composition included 0.020 mass% of fluorene and 99.756 mass% of fluorenone, and the collection rate of fluorenone by the distillation was 80.21%.

### (5. Distillation step (low boiling point component removal step))

Fluorenone containing low boiling point components obtained from the distillation column top was subjected to batch distillation to separate the low boiling point components from fluorenone using a distillation column corresponding to 9 stages. The distillation conditions were set to a pressure of 1.7 kPa, a column top temperature of 185.0°C, and a column bottom temperature of 190.0°C. Fluorenone was obtained from the distillation column bottom. The fluorenone obtained from the bottom of the distillation column was subjected to a coloring component removal step (distillation at a pressure of 1.7 kPa and a temperature of 190°C.) to obtain purified fluorenone from the top of the distillation column. The resulting purified fluorenone had a purity of 99.989%, a distillation recovery rate of fluorenone of 78.20%, and a haze of 0.11. The raw material fluorene, fluorenyl acetate, 9-methyl-9-fluorenol, and dibenzofulvene were not included in the purified fluorenone (detection limit: 0.001 mass% or less (10 ppm by mass or less)).

### Comparative Example 1

The same operation as in Example 1 was performed up to "2. Solvent removal step", so that a reaction product after solvent removal was obtained. In "3. Phosphoric acid treatment step", no phosphoric acid was added (referred to as heating step). Operations on "3. Heating step" and the followings were performed as follows.

### (3. Heating step)

The reaction product after solvent removal was introduced into a distillation column with 9 stages without addition of phosphoric acid so as to be refluxed at a pressure of 2 kPa at 245.0 to 250.0°C for 1 hour. The composition of the reaction product after the heating step included 0.06 mass% of fluorene, 82.00 mass% of fluorenone, 0.01 mass% of 9,9'-bisfluorenyl, and 0.046 mass% (460 ppm by mass) of dibenzofulvene, and fluorenyl acetate was not included therein.

### (4. Distillation step (high boiling point component removal step))

After the heating step, extraction was started under distillation conditions set to a pressure of 2 kPa, a column top temperature of 188.0°C, and a column bottom temperature of 196.0°C. From the distillation column top, the resulting fluorenone containing low boiling point components was extracted. The composition included 0.40 mass% of fluorene and 98.44 mass% of fluorenone, and 0.05 mass% of dibenzofulvene, and the collection rate of fluorenone was 76.9%.

### (5. Distillation step (low boiling point component removal step))

Fluorenone containing low boiling point components obtained from the distillation column top was subjected to batch distillation to separate the low boiling point components from fluorenone using a distillation column corresponding to 9 stages. The distillation conditions were set to a pressure of 1.7 kPa, a column top temperature of 185.0°C, and a column bottom temperature of 190.0°C. Fluorenone was obtained from the distillation column bottom. The fluorenone obtained from the bottom of the distillation column was subjected to a coloring component removal step (distillation at a pressure of 1.7 kPa and a temperature of 190°C) to obtain purified fluorenone from the top of the distillation column. The resulting purified fluorenone had a purity of 99.9%, a distillation recovery rate of fluorenone of 69.3%, and a haze of 38.4. The purified fluorenone included 0.001 mass% of raw material fluorene and 0.022 mass% (220 ppm by mass) of dibenzofulvene, and fluorenyl acetate and 9-methyl-9-fluorenol were not included therein (detection limit: 0.001 mass% or less).

**Table 1**

| | | Example 1 | Example 2 | Example 3 | Example 4 | Comparative Example 1 |
|---|---|---|---|---|---|---|
| Phosphoric acid treatment step or Heating step | Amount of phosphoric acid added | 5000ppm | 3000ppm | 4000ppm | 10000ppm | nil |
| Evaluation of fluorenone | Amount of dibenzofulvene (ppm) | nil (under detection limit) | 60 | nil (under detection limit) | nil (under detection limit) | 220 |
| | Evaluation of dullness (a value) | -12.27 | -10.23 | -12.13 | -12.33 | -10.60 |
| | Haze | 0.11 | 0.67 | 0.13 | 0.11 | 38.4 |

From the results of Examples and Comparative Examples, it is shown that according to the production method of the present invention, by-products and the like generated by the oxidation reaction can be efficiently removed, so that fluorenone having high purity and high transparency can be obtained industrially. It is also shown that according to the production method of the present invention, fluorenone having excellent appearance without dullness can be obtained.

### [Evaluation of various acids]

In "3. Phosphoric acid treatment step" of the Examples described above, the treatment was performed using various acids other than phosphoric acid instead of phosphoric acid. Example 5 (treatment with phosphoric acid)

### (1. Oxidation step and 2. Solvent removal step)

A reaction product was obtained in the same manner as in "1. Oxidation step and 2. Solvent removal step" of Example 1.

### (3. Acid treatment step)

To the reaction product after solvent removal, 5000 ppm by mass of phosphoric acid (manufactured by Fujifilm Wako Pure Chemical Corporation) (amount relative to raw material fluorene) was added, and then refluxing was performed under atmospheric pressure at 250°C for 1 hour. The resulting product (fluorenone) contained no dibenzofulvene (detection limit: 0.001 mass% or less (10 ppm by mass or less)).

### Comparative Examples 2 to 8 (treatments using various acids other than phosphoric acid)

A product (fluorenone) was obtained in the same manner as in Example 5 except that 5000 ppm by mass of phosphoric acid used in "3. Acid treatment step" in Example 5 was changed to 5000 ppm by mass of various acids shown in Table 2. The removal rate of dibenzofulvene is shown Table 2.

The removal rate (%) of dibenzofulvene was calculated as follows.

A reaction product was obtained in the same manner as in "1. Oxidation step and 2. Solvent removal step". The reaction product after solvent removal was heated under reflux under atmospheric pressure at 250°C for 1 hour without addition of acid, and the content of dibenzofulvene in the resulting product (fluorenone) was determined. In the calculation, the content of dibenzofulvene in the product (fluorenone) obtained in "3. Acid treatment step" in each of Example 5 and Comparative Examples 2 to 8 (content of dibenzofulvene after acid treatment) was divided by the content of dibenzofulvene in the product (fluorenone) obtained by heating under reflux without addition of the acid (content of dibenzofulvene after heat treatment), and expressed as a percentage. Removal rate of dibenzofulvene(%)=(Content of dibenzofulvene after acid treatment)/(Content of dibenzofulvene after heat treatment)×100

**Table 2**

| | | Example 5 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|---|
| Acid catalyst | | Phosphoric acid | Activated white clay | Iron (II) oxalate dihydrate | γ-alumina | Titanium oxide | Isophthalic acid | Sulfuric acid | Anhydrous zinc chloride |
| | Specification or the like | - | - | - | Specific surface area: 208 m²/g, | Particle size: 25 *nm or* less | - | - | - |
| | | | | | Particle size: 0.5 to 160 µm | | | | |
| | Manufacturer | FUJIFILM Wako Pure Chemical Corporation | FUJIFILM Wako Pure Chemical Corporation | FUJIFILM Wako Pure Chemical Corporation | Strem Chemicals, Inc. | Sigma-Aldrich Co. LLC | Tokyo Chemical Industry Co., Ltd. | FUJIFILM Wako Pure Chemical Corporation | Tokyo Chemical Industry Co., Ltd. |
| Removal rate of dibenzofulvene | | 100% | 0% | 23% | 60% | 18% | 89% | 48% | 0% |

From the results of Examples and Comparative Examples, it may be seen that dibenzofulvene as an impurity can be efficiently removed by treating the reaction product obtained in the oxidation step with phosphoric acid. In other words, it may be seen that according to the production method of the invention, by-products and the like generated by the oxidation reaction can be efficiently removed to obtain fluorenone having high purity.

## Claims

1. A method for producing fluorenone comprising:
an oxidation step of oxidizing fluorene in the presence of an aliphatic carboxylic acid having 2 to 3 carbon atoms, a metal catalyst, a bromine compound, and oxygen, and
a phosphoric acid treatment step of bringing an oxidation reaction mixture into contact with phosphoric acid in the order indicated.

2. The method for producing fluorenone according to claim 1, wherein the treatment temperature in the phosphoric acid treatment step is 140 to 350°C.

3. The method for producing fluorenone according to claim 1 or 2, wherein the concentration of phosphoric acid in the phosphoric acid treatment step is 3000 ppm by mass or more relative to the raw material fluorene.

4. The method for producing fluorenone according to any one of claims 1 to 3, wherein the treatment time of the phosphoric acid treatment step is 20 minutes or more.

5. The method for producing fluorenone according to any one of claims 1 to 4, further comprising a distillation step after the phosphoric acid treatment step.

6. The method for producing fluorenone according to any one of claims 1 to 5, wherein the metal catalyst is at least one selected from the group consisting of a cobalt catalyst, a manganese catalyst, a zirconium catalyst, a cerium catalyst, and a nickel catalyst.

7. The method for producing fluorenone according to any one of claims 1 to 6, wherein the aliphatic carboxylic acid is acetic acid.

8. The method for producing fluorenone according to any one of claims 1 to 7, wherein oxygen is supplied by introducing air in the oxidation step.

9. The method for producing fluorenone according to any one of claims 1 to 8, wherein the content of dibenzofulvene contained in the resulting fluorenone is 50 ppm by mass or less.

10. The method for producing fluorenone according to any one of claims 1 to 9, wherein a 20% (w/w) acetone solution of the resulting fluorenone with a thickness of 10 mm has a haze of 0.60 or less.

11. The method for producing fluorenone according to any one of claims 1 to 10, wherein the resulting fluorenone with a thickness of 25 mm has an a value of -10.30 or less at 120°C.
